# EUROPÄISCHE PATENTANMELDUNG

(11) **EP 1 679 307 A1**
(43) Veröffentlichungstag der Anmeldung: **12.07.2006**
(21) Anmeldenummer: 05100091.7
(22) Anmeldetag: 10.01.2005
(51) Int. Cl.: C07C 381/12, C01C 3/16

(54) **Herstellung und Verwendung von Sulfoniumdicyanamiden**

(71) Anmelder: Solvent Innovation GmbH, 50829 Köln (DE)
(72) Erfinder: Uerdingen, Marc, 53797 Lohmar (DE); Gerhard, Dirk, 91052 Erlangen (DE); Wasserscheid, Peter, 91054 Erlangen (DE)
(74) Vertreter: Weber, Thomas

(57) **Zusammenfassung**

Die vorliegende Erfindung betrifft ionische Flüssigkeiten der allgemeinen Formel [Sulfonium][C₂N₃], Verfahren zu deren Herstellung, deren Verwendung in Anwendungen der Elektrochemie, zur Stofftrennung und in chemischen Reaktionen. Die Erfindung betrifft ferner Farbstoffsolarzellen umfassend wenigstens ein Sulfoniumdicyanamid.

## Beschreibung

Die vorliegende Erfindung betrifft ionische Flüssigkeiten der allgemeinen Formel [Sulfonium][C₂N₃], Verfahren zu deren Herstellung, deren Verwendung in Anwendungen der Elektrochemie, zur Stofftrennung und in chemischen Reaktionen. Die Erfindung betrifft ferner Farbstoffsolarzellen umfassend wenigstens ein Sulfoniumdicyanamid.

### Technischer Hintergrund der Erfindung

Unter ionischen Flüssigkeiten versteht man im Allgemeinen Salze oder Gemische aus Salzen, deren Schmelzpunkte unterhalb 100 °C liegen (P. Wasserscheid, W. Keim, *Angew. Chem.* **2001,** *112*, 3926). Literaturbekannte Salze dieser Art bestehen aus Anionen wie z. B. Halogenostannaten, Halogenoaluminaten, Hexafluorophosphaten, Tetrafluoroboraten, Alkylsulfaten, Alkyl-oder Arylsulfonaten kombiniert mit substituierten Ammonium-, Phosphonium, Pyridinium- oder Imidazolium-Kationen. Zahlreiche Veröffentlichungen beschreiben bereits die Verwendung ionischer Flüssigkeiten als Lösungsmittel für chemische Reaktionen (T. Welton, Chem. Rev. **1999**, *99*, 2071, P. Wasserscheid, W. Keim, *Angew. Chem.,* **2000,** *112*, 3926). Weitere wichtige Einsatzfelder ionischer Flüssigkeiten liegen in ihrer Verwendung als Extraktionsmittel zur Stofftrennung (J. G. Huddleston, H. D. Willauer, R. P. Swatloski, A. E. Visser, R. D. Rogers, *Chem. Commun.* **1998**, 1765-1766; b) A. E. Visser, R. P. Swatlowski, R. D. Rogers, *Green Chemistry* **2000,** *2(1),* 1-4) sowie in ihrer Verwendung als Elektrolyt z. B. in Sensoren, Batterien, Farbstoffsolarzellen, Kondensatoren und zur elektrolytischen Abscheidung von Metallen (P. Bonhöte, A.-P. Dias, N. Papageorgiou, K. Kalyanasundaram, M. Grätzel, Inorg. Chem. 1996, 35, 1168-1178) - wobei diese Beispiele keinen Anspruch auf Vollständigkeit erheben.

Golding et al offenbaren in einer im Jahre 2002 erschienenen Publikation (J. Golding, S. A. Forsyth, G. B. Deacon, D. R. MacFarlane, Green Chemistry **2002**, *4*, 444-448) eine Reihe von Pyrollidiniumsalze sowie Tributylammoniumsalzen mit Dicyanamid-Ion und die Verbindung 1-Ethyl-3-methylimidazoliumdicyanamid. Sie beschreiben die thermischen (DSC und TGA-Analyse) sowie die elektrochemischen Eigenschaften der Verbindungen durch Veröffentlichung der Cyclovoltagramme.

Alle beschriebenen Dicyanamide wurden im Maßstab < 10g durch Umsetzung der entsprechenden Amine mit einem Alkyliodid gefolgt von einer Fällungsreaktion mit Silberdicyanamid erhalten.

Aufgrund der niedrigen Viskosität dieser Systeme im Bereich von 21 bis 50 mPa·s ist die von Golding et al beschriebene Verbindungsklasse der Dicyanamidsalze für elektrochemische Anwendungen von großem Interesse, insbesondere da diese Systeme aufgrund der hohen Anionenmobilität hohe Leitfähigkeiten aufweisen sollten.

So zeigten von Yoshida und Mitarbeitern durchgeführte Untersuchungen (Y. Yoshida, K. Muroi, A. Otsuka, G. Saito, M. Takahashi, T. Yoko, Inorg. Chem. **2004,** *43*, 1458-1462) das 1-Ethyl-3-methylimidazolium-dicyanamid mit 27 mS/cm eine sehr hohe Leitfähigkeit aufweist.

Der Erfindung liegt somit die Aufgabe zugrunde weitere ionische Flüssigkeiten mit verbesserten elektrochemischen Eigenschaften zur Verfügung zu stellen, die einen vorteilhaften Einsatz in der Technik ermöglichen.

### Beschreibung der Erfindung

Entgegen den Erwartungen des Fachmanns, dass die Leitfähigkeit von ionischen Flüssigkeiten mit abnehmender Viskosität zunimmt, wurde überraschenderweise gefunden, dass Trimethylsulfoniumdicyanamid bei einer Viskosität von 32 mPa·s eine Leitfähigkeit 25,6 mS/cm (bei einem Wassergehalt von 777 ppm (Karl Fischer Titration)) aufweist. Stattdessen muss die Viskosität des entsprechenden 1-Ethyl-3-methylimidazoliumdicyanamids zur Erzielung einer vergleichbaren Leitfähigkeit deutlich niedriger sein (Werte für die Viskosität 21 mPa·s, Leitfähigkeit 27 mS/cm, Lit. MacFarlane).

Trimethylsulfoniumdicyanamid besitzt damit überraschenderweise mit die höchste elektrische Leitfähigkeit aller ionischen Flüssigkeiten, mit Ausnahme solcher, die auf Chloroaluminatschmelzen basieren. Die Kombination aus niedriger Viskosität und hoher Leitfähigkeit ist für Anwendungen in der Stofftrennung und der Mehrphasenkatalyse, vor allem aber in elektrochemischen Anwendungen, wie z.B. als Elektrolyt/Lösungsmittel in Farbstoffsolarzellen von hohem technischem Wert.

Die Tatsache, dass durch Modifikation der Reste des Sulfonium-Kations eine Vielzahl von ionischen Flüssigkeiten mit überraschenden und technisch interessanten Eigenschaften zur Verfügung gestellt werden können wird durch zahlreiche weitere ionische Flüssigkeiten gemäß dieser Erfindung gestützt.

Gegenstand der Erfindung sind daher ionische Flüssigkeiten gemäß Formel (I) 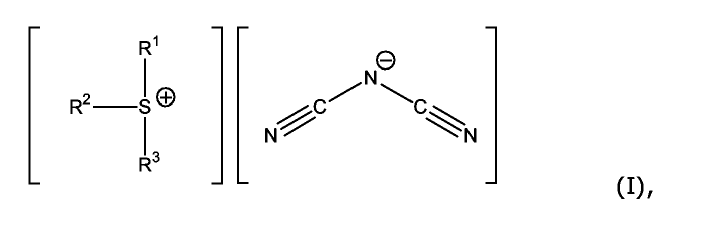 wobei die Reste R¹, R² und R³ unabhängig voneinander ausgewählt sind aus der Gruppe bestehend aus
- Wasserstoff;
- aliphatischen geradkettigen oder verzweigten Kohlenwasserstoffresten, mit 1 bis 20, vorzugsweise 2 bis 18, besonders bevorzugt 3 bis 14 Kohlenstoffatomen, die in der Kohlenstoffkette zusätzlich Heteroatome, ausgewählt aus N, S und O, sowie Unsättigungen, in Form von einer, zwei oder mehreren konjugierten oder isolierten Doppelbindungen oder einer isolierten Dreifachbindung und zudem eine oder mehrere funktionelle Gruppen aufweisen können, die ausgewählt sind aus Amino-, Carboxyl-, Acyl-, Hydroxylgruppen wobei gesättigte aliphatische Gruppen bevorzugt sind. Acylgruppen im Sinne der vorliegenden Erfindung sind Gruppen der Formel

   R⁷-C(O)-

   wobei R⁷ ausgewählt ist aus der Gruppe bestehend aus verzweigten und unverzweigten Alkylgruppen mit 1 bis 5 Kohlenstoffatomen, vorzugsweise 2 bis 4 Kohlenstoffatomen, besonders bevorzugt 3 Kohlenstoffatomen und Arylgruppen mit 5 bis 14 Kohlenstoffatomen, vorzugsweise 8 bis 10 Kohlenstoffatomen, besonders bevorzugt 6 Kohlenstoffatomen.

Bei der zuvor getroffenen Definition können naturgemäß nur Kohlenwasserstoffreste mit einer Kettenlänge von mindestens 3 Kohlenstoffatomen verzweigt sein. Besonders geeignet sind beispielsweise Kohlenwasserstoffreste, die ausgewählt sind aus der Gruppe bestehend aus Methyl, Ethyl, n-Propyl, Isopropyl, n-Butyl, sec.-Butyl, tert.-Butyl, n-Pentyl, n-Hexyl, n-Octyl oder n-Decyl.
- cycloaliphatischen Kohlenwasserstoffresten mit 3 bis 20, bevorzugt 5 bis 18, besonders bevorzugt 6 bis 8 Kohlenstoffatomen, wobei die cyclischen Reste Ring-Heteroatome, ausgewählt aus N, S und O, sowie Unsättigungen, in Form von einer, zwei oder mehreren konjugierten oder isolierten Doppelbindungen oder einer isolierten Dreifachbindung aufweisen können;
- aromatischen und araliphatischen Gruppen und mit 5 bis 22, vorzugsweise 6 bis 14, besonders bevorzugt 10 Kohlenstoffatomen im aromatischen Kern, wobei der aromatische Kern 1 bis 4, vorzugsweise 2 bis 3 Heteroatome, ausgewählt aus N, S und O, und lineare oder verzweigte Kohlenwasserstoff-Reste mit 1 bis 10, vorzugsweise 3 bis 8, besonders bevorzugt 4 bis 6 Kohlenstoffatomen aufweisen kann.

Besonders bevorzugt sind aromatische Reste, die ausgewählt sind aus der Gruppe bestehend aus Phenyl-, Naphthyl- oder Anthracylgruppen.
- Oligoethergruppen der allgemeinen Formel II

   -[(CH₂-CHR⁵)ₓ-O]_{y}-R⁴ (II),

   wobei x und y unabhängig voneinander ausgewählt sind aus ganzen Zahlen von 1 bis 250, vorzugsweise 10 und 200, besonders bevorzugt 2 bis 50 und R⁴ Wasserstoff oder eine aliphatische, cycloaliphatische, aromatische oder eine araliphatische Gruppe gemäß den vorherigen für R¹ bis R³ getroffenen Definitionen darstellt und R⁵ ein Rest, ausgewählt ist aus der Gruppe bestehend aus Wasserstoff, Methyl, Ethyl, Propyl und Butyl, vorzugsweise aus Wasserstoff und Methyl.

Besonders bevorzugt sind Oligoether auf der Basis von Ethylenoxid (EO) und/oder Propylenoxid (PO) gemäß der allgemeinen Formel III

-[(C₂H₄O)ₘ-(C₃H₆O)ₙ]-R⁴ (III),

wobei m und n unabhängig voneinander ganze Zahlen von 0 bis 250, vorzugsweise 1 bis 100, besonders bevorzugt 10 bis 50 sind und die Summe von m und n wenigstens 1 beträgt.

Im Falle von gemischten Oligoethern (m>0 und n>0) können die Ethylenoxid (EO)-Einheiten (-C₂H₄O-) und Propylenoxid (PO)-Einheiten (-C₃H₆O-) blockweise, alternierend oder statistisch verteilt sein.

Die erfindungsgemäßen ionischen Flüssigkeiten gemäß der Formel 1 werden in Folge auch als Sulfoniumdicyanamide bezeichnet.

Die erfindungsgemäßen ionischen Flüssigkeiten weisen definitionsgemäß unter Normalbedingungen (p=1013,25 mbar) einen Schmelzpunkt unterhalb 100°C auf.

Folgende Ausführungsformen sind als bevorzugt anzusehen:
- R¹, R² und R³ sind unabhängig voneinander ausgewählt aus aliphatischen geradkettigen oder verzweigten Kohlenwasserstoffresten;
- R¹, R² und R³ sind unabhängig voneinander ausgewählt aus cycloaliphatischen Kohlenwasserstoffresten;
- R¹, R² und R³ sind unabhängig voneinander ausgewählt aus aromatischen und araliphatischen Gruppen;
- R¹, R² und R³ sind unabhängig voneinander ausgewählt aus Oligoethergruppen;
- R¹ und R² sind unabhängig voneinander ausgewählt aus aliphatischen geradkettigen oder verzweigten Kohlenwasserstoffresten und R³ ist ausgewählt aus cycloaliphatischen Kohlenwasserstoffresten;
- R¹ und R² sind unabhängig voneinander ausgewählt aus aliphatischen geradkettigen oder verzweigten Kohlenwasserstoffresten und R³ ist ausgewählt aus aromatischen und araliphatischen Gruppen;
- R¹ und R² sind unabhängig voneinander ausgewählt aus aliphatischen geradkettigen oder verzweigten Kohlenwasserstoffresten und R³ ist ausgewählt aus Oligoethergruppen;
- R¹ und R² sind unabhängig voneinander ausgewählt aus aromatischen und araliphatischen Gruppen und R³ ist ausgewählt aus aliphatischen geradkettigen oder verzweigten Kohlenwasserstoffresten;
- R¹ und R² sind unabhängig voneinander ausgewählt aus aromatischen und araliphatischen Gruppen und R³ ist ausgewählt aus cycloaliphatischen Kohlenwasserstoffresten;
- R¹ und R² sind unabhängig voneinander ausgewählt aus aromatischen und araliphatischen Gruppen und R³ ist ausgewählt aus Oligoethergruppen;
- R¹ und R² sind unabhängig voneinander ausgewählt aus cycloaliphatischen Kohlenwasserstoffresten und R³ ist ausgewählt aus aliphatischen geradkettigen oder verzweigten Kohlenwasserstoffresten;
- R¹ und R² sind unabhängig voneinander ausgewählt aus cycloaliphatischen Kohlenwasserstoffresten und R³ ist ausgewählt aus aromatischen und araliphatischen Gruppen;
- R¹ und R² sind unabhängig voneinander ausgewählt aus cycloaliphatischen Kohlenwasserstoffresten und R³ ist ausgewählt aus Oligoethergruppen;
- R¹ und R² sind unabhängig voneinander ausgewählt aus Oligoethergruppen und R³ ist ausgewählt aus cycloaliphatischen Kohlenwasserstoffresten;
- R¹ und R² sind unabhängig voneinander ausgewählt aus Oligoethergruppen und R³ ist ausgewählt aus aromatischen und araliphatischen Gruppen;
- R¹ und R² sind unabhängig voneinander ausgewählt aus Oligoethergruppen und R³ ist ausgewählt aus aliphatischen geradkettigen oder verzweigten Kohlenwasserstoffresten;
- R¹ ist ein Methylalaninrest, R² und R³ sind unabhängig voneinander ausgewählt aus aliphatischen geradkettigen oder verzweigten Kohlenwasserstoffresten;
- R¹ ist Phenylmethylketonrest, R² und R³ sind unabhängig voneinander ausgewählt aus aliphatischen geradkettigen oder verzweigten Kohlenwasserstoffresten.

In einer bevorzugten Ausführungsform kann höchstens einer der Reste R¹ bis R³ Wasserstoff sein.

In besonders bevorzugten Ausführungsformen der vorliegenden Erfindung sind:
- R¹, R² R³ ausgewählt aus aliphatischen Kohlenwasserstoffresten mit einem, zwei oder drei Kohlenstoffatomen;
- R¹, R² R³ ausgewählt aus aromatischen Kohlenwasserstoffresten mit bis zu 12 Kohlenstoffatomen, vorzugsweise 10 Kohlenstoffatomen, besonders bevorzugt 6 Kohlenstoffatomen;
- R¹ und R² ausgewählt aus aliphatischen Kohlenwasserstoffresten mit einem, zwei oder drei Kohlenstoffatomen und R³ ausgewählt aus Acylverbindungen der Formel

   R⁶-C(O)-(CH₂)ₚ-

   wobei R⁶ ausgewählt ist aus der Gruppe bestehend aus verzweigten und unverzweigten Alkylgruppen mit 1 bis 5 Kohlenstoffatomen, vorzugsweise 1 bis 3 Kohlenstoffatomen, besonders bevorzugt 2 Kohlenstoffatomen und Arylgruppen mit 5 bis 14 Kohlenstoffatomen, vorzugsweise 6 bis 10 Kohlenstoffatomen, besonders bevorzugt 6 Kohlenstoffatomen; und p eine Ganze Zahl von 1 bis 6, vorzugsweise 3 bis 5, besonders bevorzugt 2 bis 4 ist.

Besonders bevorzugte ionische Flüssigkeiten gemäß dieser Erfindung sind ausgewählt aus der Gruppe bestehend aus Trimethylsulfoniumdicyanamid, Diethylmethylsulfoniumdicyanamid, Methylmethioniniumdicyanamid gemäß der nachfolgenden Formel IV, Dimethylphenylacylsulfoniumdicyanamid gemäß der nachfolgenden Formel V, Triphenylsulfoniumdicyanamid.

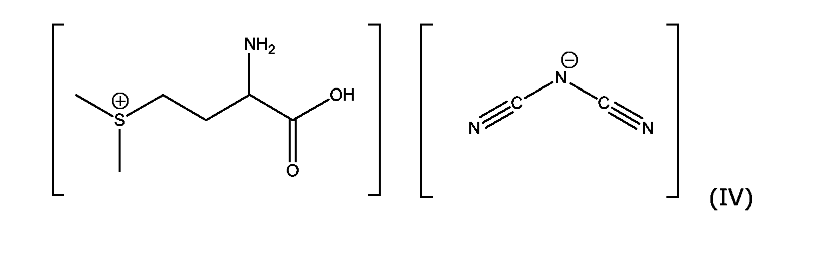

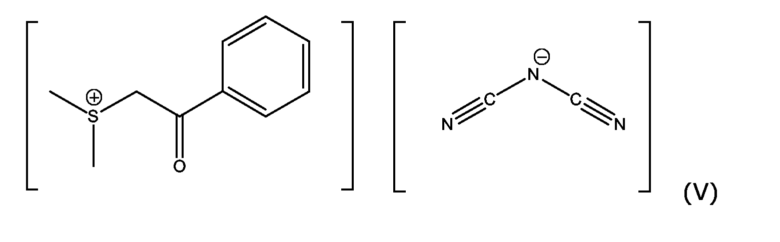

Die erfindungsgemäßen ionischen Flüssigkeiten sind gemäß den nachfolgenden Syntheserouten erhältlich:
- (i): Umsetzung eines Sulfoniumhalogenids [R¹R²R³S][X], wobei R¹ bis R³ der zuvor getroffenen Definition entsprechen und X ein Halogenid ausgewählt aus Fluorid, Chlorid, Bromid und Iodid ist, an einem mit Alkalidicyanamid, vorzugsweise Natrium- oder Kaliumdicyanamid, beladenen Ionenaustauscher.
- (ii): Umsetzung eines Sulfonium-bis(trifluoromethylsulfonyl)imids [R¹R²R³S][NTf₂], wobei R¹ bis R³ der zuvor getroffenen Definition entsprechen, mit tetra-C₁₋₈-alkylammoniumdicyanamid, vorzugsweise tetra-Butylammoniumcyanamid. Die Reaktion erfolgt vorzugsweise in einem Zweiphasensystem umfassend ein polares und ein unpolares Lösungsmittel, wie z.B. Wasser/Methylenchlorid.
- (iii): Umsetzung eines Sulfoniumhalogenids [R¹R²R³S][X], wobei R¹ bis R³ der zuvor getroffenen Definition entsprechen und X ein Halogenid ausgewählt aus Fluorid, Chlorid, Bromid und Iodid ist, mit Silberdicyanamid. In einer vorzugsweisen Ausgestaltung des erfindungsgemäßen Verfahrens können die anfallenden Silbersalze in einer ionischen Flüssigkeit gelöst und das Silber elektrolytisch abgeschieden werden.
- (iv): Umsetzung eines Sulfoniumhalogenids [R¹R²R³S][X], wobei R¹ bis R³ der zuvor getroffenen Definition entsprechen und X ein Halogenid ausgewählt aus Fluorid, Chlorid, Bromid und Iodid ist, mit Alkalidicyanamid. Die Reaktion erfolgt vorzugsweise in Aceton als Lösungsmittel.

Die erfindungsgemäßen ionischen Flüssigkeiten können einer Vielzahl von Verwendungen zugeführt werden. So eignen sie sich aufgrund ihrer Reinheit, des nicht vorhandenen Dampfdrucks und der hohen Beständigkeit als Lösungsmittel oder Lösungsmittelzusatz in chemischen Reaktionen. Die Verwendung als Lösungsmittelzusatz erfolgt in Kombination mit anderen Lösungsmitteln. Des Weiteren können die erfindungsgemäßen ionischen Flüssigkeiten als Phasentransferkatalysatoren, in mehrphasigen Reaktionssystemen als oberflächenaktive Substanzen oder Weichmacher eingesetzt werden. Außerdem eignen sich die erfindungsgemäßen ionischen Flüssigkeiten als Extraktionsmittel in Stofftrennverfahren.

Aufgrund der zuvor beschriebenen elektrochemischen Eigenschaften können die erfindungsgemäßen ionischen Flüssigkeiten insbesondere in elektrochemischen Anwendungen, wie z.B. als Elektrolyt in Batterien, Akkumulatoren, Kapazitoren oder Kondensatoren oder als Bestandteil einer Solarzelle (Lösungsmittel und/oder Elektrolyt), vorzugsweise einer Farbstoffsolarzelle oder eines Sensors Einsatz finden. In einer solchen Farbstoffsolarzelle wird als Redox-Paar eine iodidhaltige IL, vorzugsweise ein substituiertes Imidazol, wie 1-Methyl-3-propylimidazoliumiodid [PMImI], 1,2-Dimethyl-3-propyl-imidazoliumiodid [PMMImI], 1-Methyl-3-hexylimidazoliumiodid [HMImI] oder Trimethylsulfoniumiodid und elementares Iod gelöst. Als weitere Zusätze finden Basen (z.B. 4-*tert*-Butylpyridin, N-Methyl-benzimidazol, N-Methylimidazol, 1,2-Dimethyl-imidazol, Triazol) und Iodide (z.B. Lithiumiodid, Silberiodid) Verwendung. Aufgrund der hohen Leitfähigkeit, der niedrigen Viskosität und der Mischbarkeit mit Iodiden sind insbesondere Sulfoniumdicyanamide als Lösungsmittel für die Farbstoffsolarzelle geeignet.

Nach Kawano et al ergeben sich folgende optimalen Bedingungen (Kawano et al., *Journal of Photochemistry and Photobiology A: Chemistry* **2004**, *164*, 87-92) :
Verhältnis [I⁻]:[I₃-] = 10:1
Konzentration [I⁻]+[I₃⁻] = 1.0 - 2.0 mol/L
Konzentration [LiI] = 0.1 - 0.2 mol/L
Konzentration [Base] = 0.5 - 1.0 mol/L

Ein weiterer Gegenstand der vorliegenden Erfindung ist daher ein Elektrolyt für eine elektrochemische Zelle oder Solarzelle, besonders bevorzugt für eine Farbstoffsolarzelle umfassend wenigstens ein Sulfoniumdicyanamid.

Eine typische Zusammensetzung eines Elektrolyten ist:

| | |
|---|---|
| Trimethylsulfoniumdicyanamid: | 20 ml |
| 1-Methyl-3-propylimidazoliumiodid: | 1.5 mol/L |
| Iod: | 0.15 mol/L |
| 4-tert-Butylpyridin: | 0.5 mol/L |
| Lithiumiodid: | 0.1 mol/L |

Ein weiterer Gegenstand der Erfindung ist eine Batterie, ein Sensor, ein Kondensator, ein Kapazitor, eine Solarzelle oder eine Farbstoffsolarzelle umfassend wenigstens ein Sulfoniumdicyanamid.

Die vorliegende Erfindung soll anhand der folgenden Beispiele näher erläutert werden, ohne sie jedoch auf die Beispiele zu beschränken.

### Beispiele

Die Viskositäten wurden auf dem Gerät RS 100 RheoStress der Firma HAAKE im CR-Modus (Controlled Rate) gemessen. Viskositäten wurden bei einer Temperatur von 25°C bestimmt.

Die Leitfähigkeiten wurden auf dem Gerät der Firma WTW Typ LF 530, das mit der Messelektrode 980-K19/120 der Firma METTLER-TOLEDO bestückt war gemessen. Alle Messungen wurden bei 22°C durchgeführt.

Karl-Fischer-Werte wurden mittels eines METROHM 756 KF Coulometer in Kombination mit einem 832 KF Thermoprep bestimmt.

Die Anionenanalyse wurde mittels eines 761 Compact IC Ionenchromatographen der Firma METROHM mit chemischer Suppression durchgeführt.

### 1) Synthese

Für die im Folgenden beschriebenen Syntheserouten für das Produkt Trimethylsulfoniumdicyanamid wurden folgende analytischen Daten bestimmt:
0.015 molarer Ansatz: 86 % Ausbeute klare, leicht gelbliche Flüssigkeit

| NMR: | |
|---|---|
| ¹H-NMR (d⁶-DMSO): | δ = 2.84 (s, C**H**₃) ppm. |
| ¹³C-NMR (d⁶-DMSO): | δ = 26.77(**C**H₃), 119.65 (N(**C**N)₂) ppm. |
| Karl-Fischer: | 3830 ppm Wasser |
| Ionenchromatographie: | 0.5% Cl⁻ |
| | 944 ppm NO₃⁻ |
| 0.5 molarer Ansatz: | 88% Ausbeute klare, farblose Flüssigkeit |

| NMR: | |
|---|---|
| ¹H-NMR (d⁶-DMSO): | δ = 2.84 (s, C**H**₃) ppm. |
| ¹³C-NMR (d⁶-DMSO): | δ = 26.77(**C**H₃), 119.65 (N(**C**N)₂) ppm. |
| Karl-Fischer: | 777 ppm Wasser |
| Leitfähigkeit: | X²⁰ = 25.6 mS/cm. |
| Viskosität (20 °C): | η²⁰ = 32.1 mPa*s. |
| Schmelzpunkt: | -1.2 °C |

### Darstellung von Trimethylsulfoniumdicyanamid via Ionentauscher

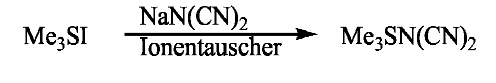

30 g Ionentauscher werden in 25 mL VE-Wasser suspendiert und in eine Säule (⌀ = 35 mm, G3-Glasfritte) gefüllt. Die Aktivierung des Ionentauschers erfolgt durch 24h-iges Spülen mit einer Lösung von Natriumdicyanamid (30 g, 0.34 mol in 250 mL VE-Wasser). Anschließend wird mit VE-Wasser (ca. 200 mL) gewaschen, bis im Eluent ein Nachweis auf Dicyanamid-Ionen mit Silbernitratlösung negativ verläuft. Der Ionentausch erfolgt durch Spülen des Ionentauschers mit einer Lösung von Trimethylsulfoniumiodid (61.21 g, 0.3 mol in 250 mL VE-Wasser). Der Eluent wird fraktioniert gesammelt. Aus jeder Fraktion wird zur Endpunktbestimmung ein Tropfen mit einer Silbernitratlösung versetzt (weißer Feststoff → Silberdicyanamid; gelber Feststoff → Silberiodid → Endpunkt überschritten). Alle wässrigen, dicyanamid-haltigen Fraktionen werden gesammelt und das Wasser unter vermindertem Druck (70 mbar, 40 °C) entfernt. Man erhält das gewünschte Produkt als eine klare, farblose Flüssigkeit.

### Darstellung von Trimethylsulfoniumdicyanamid via tetra-Butylammoniumdicyanamid

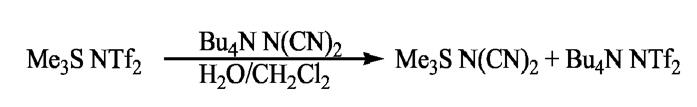

Trimethylsulfonium(bis(trifluormethylsulfonyl)imid) (35.73 g, 0.1 mol, 1.0 eq.) werden in 100 mL Dichlormethan gelöst und mit 100 mL VE-Wasser versetzt. Zu der Reaktionslösung wird tetra-Butylammoniumdicyanamid (62.71 g, 0.12 mol, 1.2 eq.) addiert und es wird für 24h bei 30 °C gerührt. Die untere, organische Phase wird im Scheidetrichter abgetrennt und dreimal mit je 20 mL VE-Wasser extrahiert. Die gesammelten wässrigen Phasen werden einmal mit 50 mL Dichlormethan extrahiert und das Wasser unter vermindertem Druck (70 mbar, 40 °C) entfernt. Man erhält das gewünschte Produkt als eine klare, farblose Flüssigkeit. Das Lösungsmittel der organischen Phase wird ebenfalls unter vermindertem Druck entfernt (850 mbar, 40 °C) entfernt, wobei ein weißer Feststoff (tetra-Butylammonium (bis(trifluormethylsulfonyl)imid)) erhalten wird.

### Darstellung von Trimethylsulfoniumdicyanamid via Silberdicyanamid

Der Anionentausch erfolgt analog zu einer Synthesevorschrift von MacFarlane (MacFarlane et al., *ChemComm* **2001**, 1430-1431).

NaN(CN)₂ (aq.) + AgNO₃ (aq.) → AgN(CN)₂ + NaNO₃ (aq.)

AgN(CN)₂ + Me₃SI → AgI + Me₃SN(CN)₂

Silbernitrat (89.39 g, 0.52 mol, 1.2 eq.) wird in 200 mL VE-Wasser gelöst und mit einer Lösung von Natriumdicyanamid (46.85 g, 0.52 mol, 1.2 eq.) in 400 mL Wasser versetzt, wobei sofort ein weißer Feststoff ausfällt. Die Reaktionsmischung wird für weitere 2h bei 25 °C gerührt. Der weiße Feststoff wird anschließend abfiltriert und dreimal mit je 300 mL gewaschen. Das Produkt, Silberdicyanamid, wird ohne weitere Trocknung in der weiteren Synthese verwendet.

Trimethylsulfoniumiodid (87.75 g, 0.43 mol, 1.0 eq.) wird in 300 mL VE-Wasser gelöst und mit Silberdicyanamid versetzt. Es fällt sofort ein gelber Feststoff aus. Es werden weitere 200 mL VE-Wasser addiert und die Reaktionsmischung für 24h bei 35 °C gerührt. Anschließend wird der gelbe Feststoff abfiltriert und zweimal mit je 100 mL VE-Wasser gewaschen. Die wässrigen Phasen werden gesammelt und das Wasser unter vermindertem Druck (70 mbar, 40°C) entfernt. Man erhält das gewünschte Produkt als eine klare, farblose Flüssigkeit. Um Spuren von Silberiodid und Trimethylsulfoniumiodid zu entfernen, wird die Flüssigkeit in Acetonitril aufgenommen und über Nacht bei 6 °C aufbewahrt. Der ausgefallene Feststoff wird abfiltriert. Das Lösungsmittel des Filtrats wird unter reduziertem Druck entfernt und das flüssige Produkt für 24h bei vermindertem Druck (40 °C, 2 mbar) getrocknet.

### Darstellung von Trimethylsulfoniumdicyanamid via Natriumdicyanamid

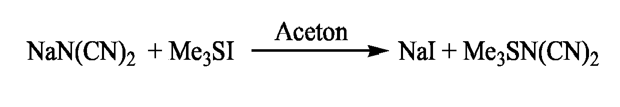

Trimethylsulfoniumiodid (10.20 g, 0.05 mol, 1.0 eq.) wird in 50 mL trockenem Aceton suspendiert und mit Natriumdicyanamid (4.45 g, 0.05 mol, 1.0 eq.) versetzt. Die weißliche Suspension wird für 4h bei Raumtemperatur gerührt. Anschließend wird der weiße Feststoff abfiltriert und das farblose Filtrat unter vermindertem Druck eingeengt. Das Produkt wird als klare, farblose Flüssigkeit erhalten. Durch Umkristallisieren mit trockenem Acetonitril wird nicht umgesetztes Edukt und Natriumiodid entfernt. Das Produkt wird für 24h bei vermindertem Druck (40 °C, 2 mbar) getrocknet.

### Elektrochemische Rückgewinnung Silbernitrat

Das bei der Synthese anfallende Silberiodid wird in einer ionischen Flüssigkeit gelöst und elektrochemisch an einer Platinelektrode abgeschieden. Nach erfolgter Elektrolyse wird das abgeschiedene Silber mit konzentrierter Salpetersäure aufgelöst. Nach Eindampfen der Lösung wird Silbernitrat als ein kristalliner, weißer Feststoff erhalten.

## Patentansprüche

1. Ionische Flüssigkeit gemäß Formel (I) 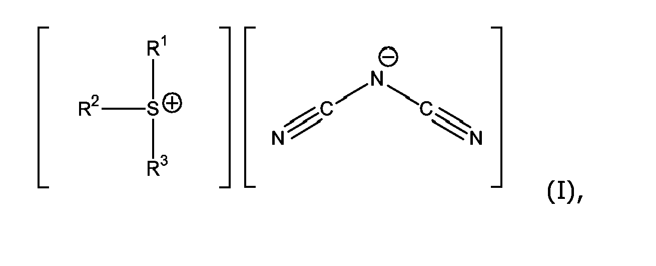 wobei die Reste R¹, R² und R³ unabhängig voneinander ausgewählt sind aus der Gruppe bestehend aus
- Wasserstoff;
- aliphatischen geradkettigen oder verzweigten Kohlenwasserstoffresten, mit 1 bis 20 Kohlenstoffatomen, die in der Kohlenstoffkette zusätzlich Heteroatome, ausgewählt aus N, S und O, sowie Unsättigungen, in Form von einer, zwei oder mehreren konjugierten oder isolierten Doppelbindungen oder einer isolierten Dreifachbindung und zudem eine oder mehrere funktionelle Gruppen aufweisen können, die ausgewählt sind aus Amino-, Carboxyl-, Acyl-, Hydroxylgruppen;
- cycloaliphatischen Kohlenwasserstoffresten mit 3 bis 20 Kohlenstoffatomen, wobei die cyclischen Reste Ring-Heteroatome, ausgewählt aus N, S und O, sowie Unsättigungen in Form von einer, zwei oder mehreren konjugierten oder isolierten Doppelbindungen oder einer isolierten Dreifachbindung aufweisen können;
- aromatischen und araliphatischen Gruppen und mit 5 bis 22 Kohlenstoffatomen im aromatischen Kern, wobei der aromatische Kern 1 bis 4 Heteroatome, ausgewählt aus N, S und O, und lineare oder verzweigte Kohlenwasserstoff-Reste mit 1 bis 10 Kohlenstoffatomen aufweisen kann;
- Oligoethergruppen der allgemeinen Formel (II)
-[(CH₂-CHR⁵)ₓ-O]_{y}-R⁴ (II),
wobei x und y unabhängig voneinander ausgewählt sind aus ganzen Zahlen von 1 bis 250 und R⁴ Wasserstoff oder eine aliphatische, cycloaliphatische, aromatische oder eine araliphatische Gruppe gemäß den vorherigen für R¹ bis R³ getroffenen Definitionen darstellt und R⁵ ein Rest, ausgewählt aus der Gruppe bestehend aus Wasserstoff, Methyl, Ethyl, Propyl und Butyl ist.

2. Ionische Flüssigkeit gemäß Anspruch 1, wobei R¹, R² und R³ unabhängig voneinander ausgewählt sind aus aliphatischen geradkettigen oder verzweigten Kohlenwasserstoffresten.

3. Ionische Flüssigkeit gemäß Anspruch 1, wobei R¹ und R² unabhängig voneinander ausgewählt sind aus aliphatischen geradkettigen oder verzweigten Kohlenwasserstoffresten und R³ ausgewählt ist aus aromatischen und araliphatischen Gruppen.

4. Ionische Flüssigkeit gemäß Anspruch 1, wobei R¹ und R² unabhängig voneinander ausgewählt sind aus aromatischen und araliphatischen Gruppen und R³ ausgewählt ist aus aliphatischen geradkettigen oder verzweigten Kohlenwasserstoffresten.

5. Ionische Flüssigkeit gemäß Anspruch 1, wobei R¹, R² und R³ unabhängig voneinander ausgewählt sind aus aromatischen und araliphatischen Gruppen.

6. Ionische Flüssigkeit gemäß irgendeinem der Ansprüche 1 bis 5, ausgewählt aus der Gruppe bestehend aus Trimethylsulfoniumdicyanamid, Diethylmethylsulfoniumdicyanamid, Methylmethioniniumdicyanamid, Dimethylphenylacylsulfoniumdicyanamid, Triphenylsulfoniumdicyanamid.

7. Ionische Flüssigkeit gemäß irgendeinem der Ansprüche 1 oder 6 und der nachfolgenden Formel IV

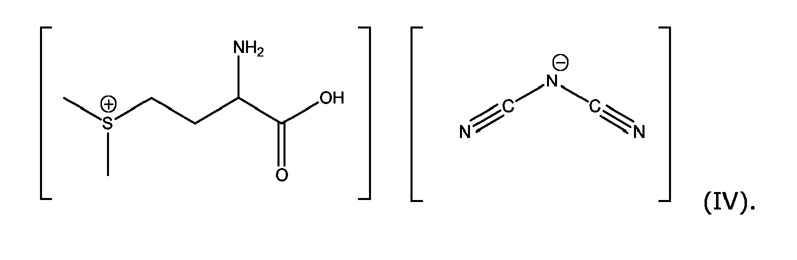

8. Ionische Flüssigkeit gemäß irgendeinem der Ansprüche 1 oder 6 und der nachfolgenden Formel V

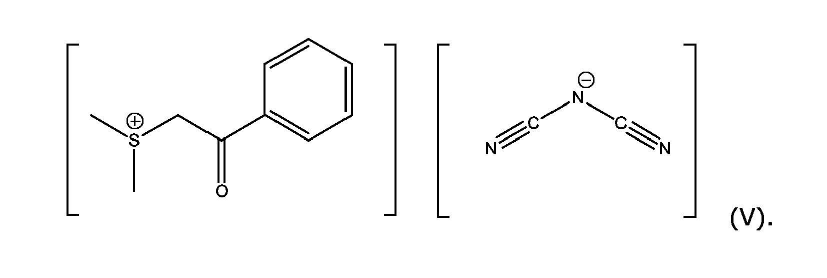

9. Verwendung der ionischen Flüssigkeiten gemäß irgendeinem der Ansprüche 1 bis 8 als Lösungsmittel oder Lösungsmittelzusatz, als Phasentransferkatalysator in mehrphasigen Reaktionssystemen, als oberflächenaktive Substanz oder Weichmacher oder als Extraktionsmittel in Stofftrennverfahren.

10. Verwendung der ionischen Flüssigkeiten gemäß irgendeinem der Ansprüche 1 bis 8 in elektrochemischen Anwendungen, Batterien, Sensoren, Kondensatoren, Kapazitoren, Solarzellen und Farbstoffsolarzellen.

11. Verfahren zur Herstellung der ionischen Flüssigkeiten gemäß irgendeinem der Ansprüche 1 bis 8 durch
(i) Umsetzung eines Sulfoniumhalogenids [R¹R²R³S][X] wobei die Reste R¹, R² und R³ unabhängig voneinander ausgewählt sind aus
- Wasserstoff;
- aliphatischen geradkettigen oder verzweigten Kohlenwasserstoffresten, mit 1 bis 20 Kohlenstoffatomen, die in der Kohlenstoffkette zusätzlich Heteroatome, ausgewählt aus N, S und O, sowie Unsättigungen, in Form von einer, zwei oder mehreren konjugierten oder isolierten Doppelbindungen oder einer isolierten Dreifachbindung und zudem eine oder mehrere funktionelle Gruppen aufweisen können, die ausgewählt sind aus Amino-, Carboxyl-, Acyl- und Hydroxylgruppen;
- cycloaliphatischen Kohlenwasserstoffresten mit 3 bis 20 Kohlenstoffatomen, wobei die cyclischen Reste Ring-Heteroatome, ausgewählt aus N, S und O, sowie Unsättigungen in Form von einer, zwei oder mehreren konjugierten oder isolierten Doppelbindungen oder einer isolierten Dreifachbindung aufweisen können;
- aromatischen und araliphatischen Gruppen und mit 5 bis 22 Kohlenstoffatomen im aromatischen Kern, wobei der aromatische Kern 1 bis 4 Heteroatome, ausgewählt aus N, S und O, und lineare oder verzweigte Kohlenwasserstoff-Reste mit 1 bis 10 Kohlenstoffatomen aufweisen kann;
- Oligoethergruppen der allgemeinen Formel (II)
-[(CH₂-CHR⁵)ₓ-O]_{y}-R⁴ (II),
wobei x und y unabhängig voneinander ausgewählt sind aus ganzen Zahlen zwischen 1 und 250 und R⁴ Wasserstoff oder eine aliphatische, cycloaliphatische, aromatische oder eine araliphatische Gruppe gemäß den vorherigen für R¹ bis R³ getroffenen Definitionen darstellt und R⁵ ein Rest, ausgewählt aus der Gruppe bestehend aus Wasserstoff, Methyl, Ethyl, Propyl und Butyl ist.
und X ein Halogenid ist, an einem mit Alkalidicyanamid beladenen Ionenaustauscher; oder
(ii) Umsetzung eines Sulfonium-bis(trifluormethylsulfonyl)imids [R¹R²R³S][NTf₂], wobei R¹ bis R³ der unter (i) getroffenen Definition entsprechen, mit einem tetra-C₁₋₈-alkylammonium-dicyanamid; oder
(iii) Umsetzung eines Sulfoniumhalogenids [R¹R²R³S][X], wobei R¹ bis R³ der unter (i) getroffenen Definition entsprechen und X ein Halogenid ist, mit Silberdicyanamid; oder
(iv) Umsetzung eines Sulfoniumhalogenids [R¹R²R³S][X], wobei R¹ bis R³ der unter (i) getroffenen Definition entsprechen und X ein Halogenid ist, mit Alkalidicyanamid.

12. Elektrolyt umfassend wenigstens eine ionische Flüssigkeit gemäß irgendeinem der Ansprüche 1 bis 8.

13. Batterie, Sensor, Kondensator, Kapazitor, Solarzelle oder Farbstoffsolarzelle umfassend wenigstens eine ionische Flüssigkeit gemäß irgendeinem der Ansprüche 1 bis 8.
